# EUROPÄISCHE PATENTANMELDUNG

(11) **EP 1 092 681 A1**
(43) Veröffentlichungstag der Anmeldung: **18.04.2001**
(21) Anmeldenummer: 99810928.4
(22) Anmeldetag: 13.10.1999
(51) Int. Cl.: C01C 1/242, C07C 67/20

(54) **Verfahren zum Neutralisieren einer Abfallsäure und gleichzeitigem Gewinnen von Salzkristallen**

(71) Anmelder: Va Tech Wabag AG, 8401 Winterthur (CH)
(72) Erfinder: Weilenmann, Max, 8037 Zürich (CH); Ainscow, Stuart, 8400 Winterhur (CH)
(74) Vertreter: VA TECH Patente GmbH

(57) **Zusammenfassung**

Das Verfahren dient dazu, eine Abfallsäure (S) zu neutralisieren und gleichzeitig Salzkristalle (AS) der beim Neutralisieren gebildeten Salze zu gewinnen. Insbesondere ist das Verfahren dazu geeignet, Ammoniumsulfat (AS) aus einer Abfallschwefelsäure (S) unter Verwendung von Ammoniak als Neutralisierungsmittel (A) herzustellen. Die Abfallsäure ist eine wässrige, mit organischen Stoffen (OG) befrachtete Lösung. Reaktionswärme, die beim Neutralisieren freigesetzt wird, lässt sich zu einer Aufkonzentrierung der Lösung und dem Kristallisieren nutzen. In einem ersten Teilverfahren (I) wird die Abfallsäure neutralisiert sowie - zwecks Vermeidung einer Kristallisation des Salzes - mit Wasser (W) verdünnt und es werden organische Stoffe, die sich beim Neutralisieren aus der wässrigen Lösung (L) absondern, entfernt. Aus der wässrigen Lösung (L) wird in einem zweiten Teilverfahren (II) mittels eines Verdampfungs/Kristallisations-Verfahrens unter Nutzung der im ersten Teilverfahren freigesetzten Reaktionswärme ein Kristallisat (C) aus Kristallen des Salzes - insbesondere Ammoniumsulfat - erzeugt und als Produkt (AS) abgezogen.

## Beschreibung

Die Erfindung betrifft ein Verfahren zum Neutralisieren einer Abfallsäure und gleichzeitigem Gewinnen von Salzkristallen gemäss Oberbegriff von Anspruch 1 sowie eine Anwendung des Verfahrens.

Bei einem bekannten Verfahren zur Erzeugung von Methylmethacrylat MMA, dem Monomer von PMMA, lässt man eine Säure, nämlich Methacrylamidsulfat, mit Methanol unter einer Esterbildung zu MMA reagieren. Dabei muss das MMA durch Destillation von einer Abfallschwefelsäure getrennt werden, die eine wässrige, mit organischen Stoffen befrachtete Lösung ist. Indem man diese Abfallsäure mit Ammoniak NH₃ neutralisiert, kann man Ammoniumsulfat (NH₄)₂SO₄ als Stickstoffdünger für die Landwirtschaft gewinnen. Da die Neutralisierungsreaktion stark exotherm ist, kann die freigesetzte Reaktionswärme genutzt werden, um die Lösung durch Abgabe von Wasserdampf aufkonzentrieren und das Ammoniumsulfat teilweise auskristallisieren zu lassen. Unter einer zusätzlichen Wärmezufuhr ergibt dieses Verdampfungs/Kristallisations-Verfahren ein Gemisch aus Kristallisat und Flüssigkeit, aus dem das Kristallisat durch Zentrifugieren separierbar ist. Bei einem wirtschaftlichen Verfahren wird für die zusätzliche Wärmezufuhr 0,18 kg Dampf für 1 kg Abfallsäure benötigt. Da das erzeugte Kristallisat noch relativ stark verunreinigt ist, hat man vorgeschlagen, mittels eines nochmaligen Auflösens und einer zweiten Kristallisation die Qualität des Produkts zu verbesseren. Dabei wäre allerdings, um den Energiebedarf für die zweite Kristallisation decken zu können, zusätzlich 0,54 kg Dampf bezogen auf 1 kg Abfallsäure erforderlich.

Aufgabe der Erfindung ist es, ein Verfahren zu schaffen, bei dem a) eine Neutralisierung einer Abfallsäuren mit einer Gewinnung von Kristallen der gebildeten Salze gekoppelt ist und b) aus der neutralisierten Abfallsäure, die eine wässrige, mit organischen Stoffen befrachtete Lösung ist, ein Kristallisat erzeugbar ist, das bezüglich einer Verunreinigung mit den organischen Stoffen eine bessere Qualität aufweist als das genannte Verfahren mit nur einer Kristallisation. Ausserdem soll c) für dieses Verfahren wesentlich weniger Energie als für das Verfahren mit zwei Kristallisationen benötigt werden. Diese Aufgabe wird durch das im Anspruch 1 definierte Verfahren gelöst.

Das Verfahren dient dazu, eine Abfallsäure zu neutralisieren und gleichzeitig Salzkristalle der beim Neutralisieren gebildeten Salze zu gewinnen. Insbesondere ist das Verfahren dazu geeignet, Ammoniumsulfat aus einer Abfallschwefelsäure unter Verwendung von Ammoniak als Neutralisierungsmittel herzustellen. Die Abfallsäure ist eine wässrige, mit organischen Stoffen befrachtete Lösung. Reaktionswärme, die beim Neutralisieren freigesetzt wird, lässt sich zu einer Aufkonzentrierung der Lösung und dem Kristallisieren nutzen. In einem ersten Teilverfahren wird die Abfallsäure neutralisiert sowie - zwecks Vermeidung einer Kristallisation des Salzes - mit Wasser verdünnt und es werden organische Stoffe, die sich beim Neutralisieren aus der wässrigen Lösung absondern, entfernt. Aus der wässrigen Lösung wird in einem zweiten Teilverfahren mittels eines Verdampfungs/Kristallisations-Verfahrens unter Nutzung der im ersten Teilverfahren freigesetzten Reaktionswärme ein Kristallisat aus Kristallen des Salzes - insbesondere Ammoniumsulfat - erzeugt und als Produkt abgezogen.

Die Ansprüche 2 bis 9 betreffen vorteilhafte Ausführungsformen des erfindungsgemässen Verfahrens nach Anspruch 1. Gegenstand des Anspruchs 10 ist eine Anwendung des Verfahrens.

Nachfolgend wird die Erfindung anhand der Zeichnungen erläutert. Es zeigen:
- Fig. 1: ein das erfindungsgemässe Verfahren darstellendes Blockschema,
- Fig. 2: einen Reaktor zum Durchführen eines ersten Teilverfahrens,
- Fig. 3: eine Verdampfungs/Kristallisations-Einrichtung für ein zweites Teilverfahren,
- Fig. 4: eine symbolische Darstellung der Einrichtung gemäss Fig. 3 und
- Fig. 5: ein Schema zu einem zweistufig durchgeführten zweiten Teilverfahren.

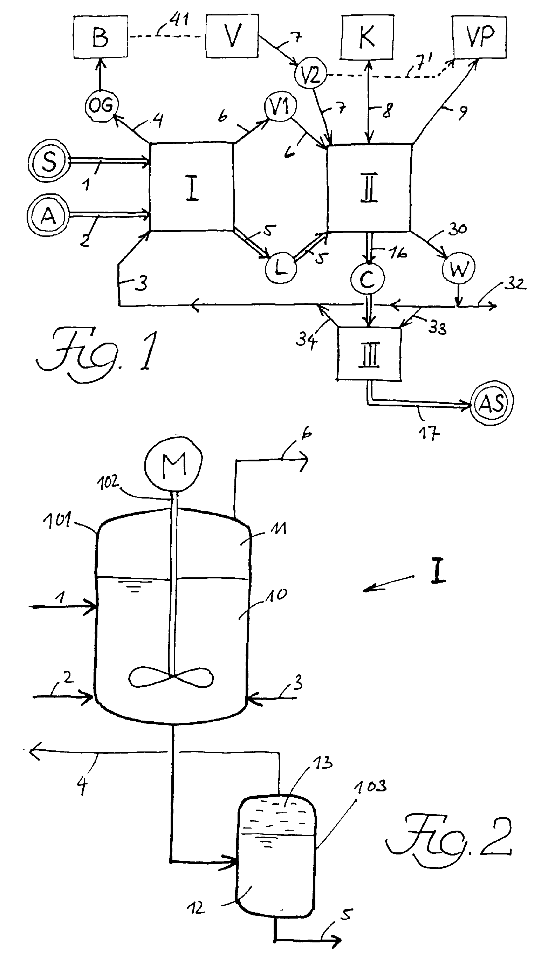

Das in Fig. 1 dargestellte erfindungsgemässe Verfahren umfasst drei Teilverfahren I, II und III. Im ersten Teilverfahren I wird eine Abfallsäure S und ein Neutralisierungsmittel A durch Leitungen 1 bzw. 2 in einen Reaktor eingespeist. Aufgrund einer freigesetzten Reaktionswärme geht Wasser der Abfallsäure S in Dampf V1 über, der über eine Leitung 6 in das zweite Teilverfahren II geleitet wird.

Es ist sinnvoll, die beiden Teilverfahren I und II getrennt zu führen, da organische Stoffe OG der Abfallsäure S sich beim Neutralisieren aus der wässrigen Lösung L absondern. Diese abgesonderten Stoffe, die im Fall der MMA-Abfallsäure eine Phase bilden, die leichter als Wasser ist, lassen sich von der Lösung L abtrennen. Für eine gute Abtrennung ist es erforderlich, dass keine Kristalle in der Lösung L enthalten sind. Daher wird zusätzlich zum Neutralisierungsmittel A auch Wasser W der Abfallsäure S zugemischt, um zu verhindern, dass bereits im Teilverfahren I Salzkristalle entstehen. Die Tatsache, dass organische Stoffe während des Neutralisierens in einer abtrennbaren Form abgesondert werden, ist anscheinend eine neue Erkenntnis, die in der vorliegenden Erfindung genutzt wird.

Die organischen Stoffe OG werden von der Lösung L abgetrennt und über eine Leitung 4 beispielsweise einer Verbrennung B zugeführt. Die Lösung L gelangt über eine Leitung 5 in eine Verdampfungs/Kristallisations-Einrichtung (kurz Verdampfer/Kristallisator) des zweiten Teilverfahrens II, wo eine Kristallisation mittels eines ein- oder mehrstufigen Verfahrens durchgeführt wird. Diese Einrichtung ist über eine Leitung 9 an eine Vakuumpumpe VP angeschlossen, mit der Inertgase und flüchtige organische Stoffe aus dem Verfahren entfernt werden. Bei dem Teilverfahrens II wird zusätzlicher Dampf V2 benötigt, der mit einem Dampferzeuger V erzeugt und über eine Leitung 7 zugeführt wird. Abwärme 41 aus der Verbrennung B kann im Verdampfer genutzt werden. Dies ist mit der gestrichelt gezeichneten Linie 41 angedeutet. Ein Teil des Dampfes V2 kann auch für nicht dargestellte Ejektoren der Vakuumpumpe VP zur Erzeugung von Treibstrahlen verwendet werden.

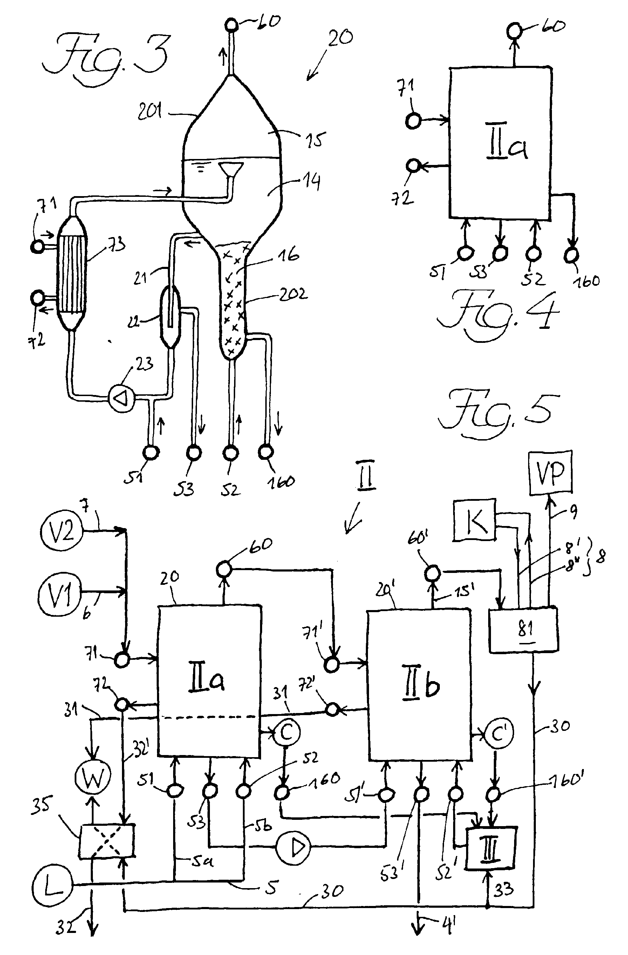

Dampf, der im Verdampfer/Kristallisator entsteht, wird mittels einer Kühleinrichtung K durch eine Wärmeabfuhr 8 auskondensiert und als Wasser W über eine Leitung 30 abgezogen. Ein Teil des Wassers W wird in das Verfahren rückgeführt, der Rest - Leitung 32 - aus dem Verfahren entfernt. (Bei einem nachfolgend anhand der Fig. 5 erläuterten Verfahren sind die Verhältnisse etwas komplizierter.) Kristallisat C, das im Teilverfahren II gewonnen worden ist, gelangt über eine Leitung 16 in das dritte Teilverfahren III, wo es zu einem nutzbaren Produkt AS aufbereitet wird. Durch Zentrifugieren und gleichzeitiges Waschen (Waschwasser 33) können Verunreinigungen zu einem weiteren Teil aus dem Kristallisat C entfernt werden. Gebrauchtes Waschwasser wird - Pfeil 34 - in das Verfahren rückgeführt. Das Produkt C kann schliesslich durch Trocknen in einem Fliessbett zu einem rieselfähigen Kristallisat AS aufbereitet und über eine Leitung 17 seinem Verwendungszweck zugeführt werden.

Fig. 2 zeigt einen Reaktor zum Durchführen des ersten Teilverfahrens I. Die Abfallsäure S, das Neutralisierungsmittel A sowie Wasser W werden - Pfeile 1, 2 bzw. 3 - in einen ersten Behälter 101 eingespeist, wo die Neutralisierung unter Rühren mittels eines Rührwerks 102 durchgeführt wird. Eine flüssige Phase 10 ist heterogen; sie besteht aus einer wässrigen Lösung und abgesonderten organischen Stoffen OG. Freigesetzter Wasserdampf bildet eine Dampfphase 11, aus der über die Leitung 6 der Dampf V1 abgezogen und dem zweiten Teilverfahren II zugeführt wird. Die abgesonderten organischen Stoffe OG werden in einem zweiten Behälter 103 als Phase 13 von einer Phase 12 separiert und über die Leitung 4 entfernt. Die Phase 12 ist die wässrige Lösung L, die über die Leitung 5 dem zweite Teilverfahren II zugeführt wird.

Fig. 3 zeigt eine Verdampfungs/Kristallisations-Einrichtung 20 mit einem Behälter 201 für ein zweites Teilverfahren II. In einem sogenannten Magma 14, das aus einer flüssigen Phase und in dieser suspendierten Kristallen gebildet wird, wachsen und vermehren sich die Kristalle. Dem Magma 14 wird in einem Zwangskreislauf 21 zu behandelnde Lösung - über einen Eingang 51 - sowie die für die Kristallisation benötigte Wärme - indirekt mittels eines Heizdampfes, in einem Wärmetauscher 73 - zugeführt. Das Magma 14 gelangt zuerst in einen Separator 22, aus dem flüssige Phase entnehmbar ist, um organische Verunreinigungen, die sich in der Einrichtung 20 aufkonzentrieren, über einen Ausgang 53 entfernen zu können. Eine Pumpe 23 fördert das Magma 14 zu dem als Kondensator ausgebildeten Wärmetauscher 73, der einen Eingang 71 für den Heizdampf und einen Ausgang 72 für das Kondensat aufweist. Das erwärmte Magma gibt Dampf in die Dampfphase 15 ab, der über einen Ausgang 60 aus der Einrichtung 20 abziehbar ist. Der Behälter 201 weist an seiner tiefsten Stelle eine rohrförmige Absetzstelle 202 für das Kristallisat 16 auf. Über einen Eingang 52 am Fuss des Behälters kann Flüssigkeit zugeführt werden, mit der sich das Kristallisat 16 im Gegenstrom waschen lässt. Das gewonnene Kristallisat C wird über einen Ausgang 160 aus der Einrichtung 20 abgezogen.

Fig. 4 zeigt eine symbolische Darstellung der Einrichtung 20 in Form eines Blocksymbols IIa mit Eingängen 51, 52, 71 und Ausgängen 53, 60, 160 sowie 72, deren Bedeutung sich anhand der Fig.3 ablesen lässt.

Die Verdampfung/Kristallisation des zweiten Teilverfahrens II kann mehrstufig unter Nutzung eines Multieffekts durchgeführt werden, bei dem - für jede Stufe mit Ausnahme der letzten - Dampf, der durch die Stufe abgegeben wird, unverdichtet in der nachfolgenden Stufe als Wärmequelle genutzt wird. Fig. 5 zeigt ein zweistufiges Verfahren, das unter Verwendung des Blocksymbols IIa gemäss Fig. 4 dargestellt ist. Das Blocksymbol IIb mit den Eingängen 51', 52', 71' und Ausgängen 53', 60', 160', 72' stellt im wesentlichen eine gleiche Einrichtung 20' wie das Blocksymbol IIa dar, die lediglich auf eine andere Art im Gesamtsystem integriert ist. In der dem Blocksymbol IIb entsprechenden Stufe, kurz Stufe IIb, wird ein Kristallisat C' erzeugt.

Aufgrund der Erläuterungen zu den Figuren 1 und 3 lässt sich das in Fig. 5 dargestellte Teilverfahren II weitgehend verstehen, ohne dass auf alle Einzelheiten eingegangen werden muss. Es sind noch ergänzende Kommentare nötig:

Das dargestellte Verfahren ist für die Herstellung von Ammoniumsulfat aus MMA-Abfallsäure entwickelt worden, bei der beispielsweise pro Stunde 11 Tonnen Abfallsäure mit rund 1,6 t/h Ammoniak neutralisiert wird. Die Abfallsäure enthält rund 1 Gew.% organische Stoffe OG. Die neutralisierte und weiter zu behandelnde Lösung L - rund 20 t/h - wird über die Leitung 5 in die Einrichtung 20 eingespeist und zuvor in zwei Teilströme 5a und 5b verzweigt. Der eine Teilstrom 5b wird für das Waschen des Kristallisats 16 verwendet. Kondensat 32' des Heizdampfes, der ein Gemisch von V1 und V2 ist, rund 3 bzw. 2 t/h, hat eine Temperatur von 89°C und kann daher in einem Wärmetauscher 35 zur Erwärmung von Kondensat 30 aus der zweiten Stufe IIb genutzt werden. Das abgekühlte Kondensat 32 wird aus dem Verfahren enfernt. Der Anteil der Lösung L, dem das Ammoniumsulfat durch die Kristallisation weitgehend entzogen worden ist, wird über den Ausgang 53' der zweiten Stufe IIb und Leitung 4' aus dem Verfahren enfernt. Er enhält organische Stoffe, die sich zusätzlich im zweiten Teilverfahren II haben abtrennen lassen, so dass für das Produkt AS eine wesentlich grössere Reinheit als bei dem bekannten Kristallisationsverfahren erzielbar ist.

Wasser W, das beim Neutralisieren zum Verdünnen verwendet wird, setzt sich aus dem erwärmten Kondensat 30 und einem Kondensat 31 zusammen, das in der zweiten Stufe IIb aus Dampf 15 (siehe Fig. 3) verflüssigt worden ist. Der Dampf 15 aus der Stufe IIa hat dort eine Temperatur von 72°C. Dampf 15' der zweiten Stufe IIb (51°C) wird in einem Kondensator 81 zu dem Kondensat 30 verflüssigt. Dieser Kondensator 81 ist über zwei Kühlwasserleitungen 8' und 8" an die Kühleinrichtung K angeschlossen. Über die Verbindung 9 zwischen dem Kondensator 81 und der Vakuumpumpe VP werden nicht kondensierbare Gase aus dem Verfahren entfernt. Ein Teil des Kondensats 30 kann bei einer Zentrifugierung im Teilverfahren III als Waschwasser verwendet. Zu diesem Zweck kann auch frisches Wasser aus einem externen Wassernetz verwendet werden. Das gebrauchte Waschwasser wird in der Einrichtung 20' über den Eingang 52' eingespeist und für ein Gegenstromwaschen des Kristallisats 16' noch einmal genutzt. Das gewonnene Kristallisat C' wird über den Ausgang 160' zur Aufbereitung in das Teilverfahren III weitergeleitet.

Im Zwangskreislauf 21 der ersten Stufe IIa wird die umgewälzte Flüssigkeit auf eine Temperatur zwischen 65 und 90°C, vorzugsweise 80°C, erwärmt. Die entsprechende Temperatur der zweiten Stufe IIb hat einen Wert zwischen 45 und 70°C, vorzugsweise 61°C.

Der Energiebedarf für das zweistufige Verfahren beträgt 0,19 kg Dampf bezogen auf 1 kg Abfallsäure, wenn für die Temperaturen die als bevorzugt angesehenen Werte gewählt werden. Er ist somit praktisch gleich gross wie der Energiebedarf beim bekannten Kristallisationsverfahren. Bei einem einstufigen Verfahren wäre der Energiebedarf doppelt so gross. Der Energiebedarf lässt sich weiter senken, wenn zu einem Verfahren mit drei oder mehr Stufen übergegangen wird. Allerdings nimmt dabei gleichzeitig der apparative Aufwand zu, so dass ein vielstufiges Verfahren wirtschaftlich kaum mehr sinnvoll ist.

Eine weitere Möglichkeit, den Energiebedarf zu reduzieren, besteht darin, dass man einen Wärmepumpeneffekt nutzt. So kann beispielsweise die Verdampfung des zweiten Teilverfahrens II und die für die Verdampfung erforderliche Wärmezufuhr mittels einer Brüdenverdichtung gekoppelt werden, indem der Dampf 15 der Einrichtung 20 mechanisch verdichtet wird und der so verdichtete Dampf über den Eingang 71 in den Kondensator 73 eingespeist wird.

## Patentansprüche

1. Verfahren zum Neutralisieren einer Abfallsäuren (S) und gleichzeitigem Gewinnen von Salzkristallen (AS) der beim Neutralisieren gebildeten Salze, insbesondere zum Herstellen von Ammoniumsulfat (AS) aus einer Abfallschwefelsäure (S) unter Verwendung von Ammoniak als Neutralisierungsmittel (A), wobei die Abfallsäure eine wässrige, mit organischen Stoffen (OG) befrachtete Lösung ist und Reaktionswärme, die beim Neutralisieren freigesetzt wird, zu einer Aufkonzentrierung der Lösung und dem Kristallisieren genutzt wird, dadurch gekennzeichnet, dass in einem ersten Teilverfahren (I) die Abfallsäure neutralisiert sowie - zwecks Vermeidung einer Kristallisation des Salzes - mit Wasser (W) verdünnt wird und organische Stoffe, die sich beim Neutralisieren aus der wässrigen Lösung (L) absondern, entfernt werden und
dass aus der wässrigen Lösung (L) in einem zweiten Teilverfahren (II) mittels eines Verdampfungs/Kristallisations-Verfahrens unter Nutzung der im ersten Teilverfahren freigesetzten Reaktionswärme ein Kristallisat (C) aus Kristallen des Salzes, insbesondere des Ammoniumsulfats, erzeugt und als Produkt (AS) abgezogen wird.

2. Verfahren nach Anspruch 1, dadurch gekennzeichnet, dass die Neutralisierung unter Rühren in einem ersten Behälter (201) durchgeführt wird, aus dem freigesetzter Wasserdampf (V1) abgezogen und dem zweiten Teilverfahren (II) zugeführt wird, und dass die abgesonderten organischen Stoffe (OG) in einem zweiten Behälter (103) separiert werden.

3. Verfahren nach Anspruch 1 oder 2, dadurch gekennzeichnet, dass die für die Kristallisation benötigte Wärme einem Verdampfer/Kristallisator (20) indirekt mittels Dampf (V1, V2) zugeführt wird, wobei der Dampf ausser mit dem ersten Teilverfahren (I) zusätzlich mit einem Dampferzeuger (V) bereitgestellt wird.

4. Verfahren nach einem der Ansprüche 1 bis 3, dadurch gekennzeichnet, dass die Verdampfung/Kristallisation (20) des zweiten Teilverfahrens (II) mehrstufig unter Nutzung eines Multieffekts durchgeführt wird, bei dem - für jede Stufe (IIa) mit Ausnahme der letzten - Dampf (15), der durch die Stufe abgegeben wird, unverdichtet in der nachfolgenden Stufe (IIb) als Wärmequelle genutzt wird

5. Verfahren nach Anspruch 4, dadurch gekennzeichnet, dass Ammoniumsulfat (AS) aus Abfallschwefelsäure (S) hergestellt wird, dass die Verdampfung/Kristallisation (II) zweistufig durchgeführt wird, dass für die Temperaturen in der ersten Stufe (IIa) Werte zwischen 65 und 90°C, vorzugsweise bei 80°C, und in der zweiten Stufe (IIb) Werte zwischen 45 und 70°C, vorzugsweise bei 61 °C, eingestellt werden.

6. Verfahren nach einem der Ansprüche 1 bis 3, dadurch gekennzeichnet, dass die Verdampfung des zweiten Teilverfahrens (II) und die für die Verdampfung erforderliche Wärmezufuhr mittels einer Brüdenverdichtung gekoppelt werden.

7. Verfahren nach einem der Ansprüche 1 bis 5, dadurch gekennzeichnet, dass für das zweite Teilverfahren (II) Behälter (201) verwendet werden, die an ihren tiefsten Stellen jeweils eine rohrförmige Absetzstelle (202) für das Kristallisat aufweisen, in welcher das sich absetzende Kristallisat (16) im Gegenstrom waschbar ist.

8. Verfahren nach einem der Ansprüche 1 bis 7, dadurch gekennzeichnet, dass in einem dritten Teilverfahren das Kristallisat (C, C') zu einem nutzbaren Produkt (AS) aufbereitet wird, insbesondere durch Zentrifugieren und gleichzeitiges Waschen.

9. Verfahren nach Anspruch 8, dadurch gekennzeichnet, dass das Produkt (AS) zu einem rieselfähigen Kristallisat aufbereitet wird, insbesondere durch Trocknen in einem Fliessbett.

10. Anwendung des Verfahrens gemäss einem der Ansprüche 1 bis 9 zur Erzeugung von als Stickstoffdünger verwendbarem Ammoniumsulfat (AS), wobei eine Abfallschwefelsäure (S), die bei einer Erzeugung von MMA entstanden ist, genutzt wird.
